# EUROPÄISCHE PATENTANMELDUNG

(11) **EP 2 363 059 A1**
(43) Veröffentlichungstag der Anmeldung: **07.09.2011**
(21) Anmeldenummer: 10002065.0
(22) Anmeldetag: 01.03.2010
(51) Int. Cl.: A61B 3/032, A61B 3/036

(54) **Simultane Netzhautbilder unterschiedlicher Bildweite zur subjektiven Refraktion**

(71) Anmelder: Stuetz, Ignaz Alois, 4212 Neumarkt im Muehlkreis (AT)
(72) Erfinder: Stuetz, Ignaz Alois, 4212 Neumarkt im Muehlkreis (AT)
(74) Vertreter: Piermayr, Alexander

(57) **Zusammenfassung**

Bei subjektiven Brillenglasbestimmungen werden Probanden Messgläser mit verschiedenen refraktiven Wirkungen vorgehalten oder geschaltet. Sie müssen zeitlich hintereinander die Qualität der Scheindrücke vergleichen und entscheiden, welcher davon besser ist. Ohne das ganze Testbild zu verdoppeln erlaubt die Erfindung durch entsprechende Anordnung optisch-mechanisch-elektronischer Elemente die gleichzeitige Generierung subjektiv neben- oder übereinander angeordneter visueller Objekte, die aber verschiedene refraktive Wirkungen erfahren, das heißt, dass die Abbildungen unterschiedliche Bildlagen entlang der optischen Achse, näher bzw. weiter weg von der Netzhaut, aufweisen. Damit können Probanden zwei oder auch mehrere visuelle Objekte gleichzeitig neben- oder Übereinander beobachten und mit geringer Verwechslungsgefahr auch motorisch angeben, welches davon die beste Abbildungsqualität aufweist. Prinzipiell für die monokulare subjektive Brillenglasbestimmung konzipiert ist die Erfindung beliebig mit binokularen Verfahren kombinierbar, sodass damit zügiger die beste Korrektion in entspannt-kommunikativer Atmosphäre ermittelt werden kann - ein wesentlicher Fortschritt bei komplexen Refraktionsdefiziten sowie bei der Arbeit mit Kindern, sehschwachen oder retardierten Personen.

## Beschreibung

### 1. Das technische Gebiet:

In der Optometrie erfolgt die optimierte Augenglasbesbestimmung durch Kommunikation mit dem Probanden, der seine subjektiven Eindrücke auf ihm nacheinander gestellte Sehaufgaben wiedergibt.

### 2. Stand der Technik:

Bei den gängigen subjektiven Verfahren zur Augenglashestimmung werden die Reaktionen und Antworten der Probanden auf die gestellten Sehaufgaben verwertet. Es werden optische Elemente verwendet, mit denen unterschiedliche refraktive Wirkungen (sphärisch-cylindrisch-Achse-Prisma) hergestellt werden, wobei die Qualität der Seheindrücke von den Probanden zu vergleichen ist. Diese sind gängig so angeordnet, dass zeitlich hintereinander zwei in der Brechkraft = Refraktion unterschiedliche Varianten präsentiert werden, wonach der Proband angeben muss, ob der Erste oder der Zweite subjektiv einen kontrastreicheren Eindruck vermittelt.

Probanden haben also die Aufgabe, die Qualität von Seheindrücken aufgrund der gängigen technischen Ausführung hintereinander mit minimalen Unterschieden subjektiv zu beurteilen. Das kommt im Alltag praktisch nie vor, sodass die Probanden keine Übung darin haben und oft verunsichert sind. Dazu kommt die Notwendigkeit, sich die Qualität des ersten Seheindruckes zu merken und mit der Abbildungsqualität eines zweiten zu vergleichen. Daher müssen die Varianten "eins" und "zwei" mehrmals vom Prüfer vorgeführt werden; Verwechslungen sind in der Praxis nicht vermeidbar und verlangen vom Prüfer einen hohe Konzentration sowie, wenn auch nur der Verdacht auf ein Missverständnis besteht, zusätzlichen Zeitaufwand zur Klärung.

Ein zweites Problem der gängigen subjektiven Verfahren ist die vom Prüfer vorgegebene Zeit, in welcher der jeweilige Seheindruck betrachten werden darf. Diese wird von den Probanden meist als zu kurz empfunden. Zu beachten ist drittens, dass insbesondere junge Menschen durch die Akkommodationstähigkeit sehr rasch die Einstellrefraktion des Auges ändern können, sodass die zeitlich hintereinander präsentierten Varianten gleich gut erscheinen und damit subjektiv kein Unterschied angegeben werden kann. - Ein klassisches Dilemma: die Probanden möchten mehr Zeit zum Betrachten, die Prüfer wollen und müssen aus den genannten Gründen die Präsentation so kurz wie möglich halten.

Ein simultaner Vergleich von unterschiedlichen Seheindrücken hätte also viele Vorteile, auf die später noch detailliert eingegangen werden soll.

### 3. Beschreibung der Erfindung im Detail:

Anstatt der bisher gängigen Anordnung der optischen Elemente, die nur die Darstellung zeitlich hintereinander folgender Variationen der Brillenglaskorrektion zulassen, ist zentraler Punkt der vorliegenden Erfindung die zeitlich simultane Vergleichsmöglichkeit, dass also gleiche oder ähnliche Sehobjekte an vertikal oder horizontal nahe beieinanderliegenden Orten mit unterschiedlichen Refraktionswirkungen, also verschiedenen Bildlagen entlang der optischen Achse auf, vor oder hinter die Netzhaut, abgebildet werden.

Beim Schsinn gibt es einen wesentlichen Unterschied zwischen dem verbreiteten Verständnis und Sprachgebrauch einerseits und den physikalisch-physiologischen Vorgängen andererseits. Während also das Sehen tatsächlich ein passiver, sensorischer Vorgang ist, indem Licht auf die Netzhaut fällt, in Nervenimpulse umgewandelt und im Gehirn interpretiert wird, ist umgangssprachlich das "Sehen" eine Aktion: der Mensch blickt elwas an. Bei dieser gebräuchlichen Betrachtungsweise geht vom Menschen ein "Sehstrahl" aus, der bei Anwendung von optischen Vorrichtungen als "Beobachtungsstrahlengang" unterschiedlich gebrochen und in verschiedene Teile aufgespalten werden kann. Für die nachfolgende Beschreibung wurde die tatsächliche, physikalisch-physiologische Perspektive gewählt, bei welcher von Objekten Licht ausgeht, das verschiedene Abbildungsstrahlengänge und Refraktionen durchlaufend so zusammengeführt wird, dass die Objekte auf der Netzhaut nebeneinander mit verschiedenen Bildlagen abgebildet werden. Je näher diese Bildlage der Netzhaut ist, umso besser wird die subjektive Abbildungsqualität sein.

Es geht im Folgenden, auch wenn des öfteren von Strahlenteilung, Trenner, etwa durch Polarisation und Zusammenführung von Strahlengänge die Rede sein wird, prinzipiell um eine neue technische Anordnung optisch-mechanisch-elektronischer Elemente zur Durchführung eines subjektiven, monokularen Verfahrens zur Brillenglasbestimmung. Dessenungeachtet kann es beliebig mit binokularen Prüf- und Optimierungsmethoden kombiniert werden. Diese Kombinationsmöglichkeit ist aber nicht der zentrale Punkt der Erfindung.

(Anmerkung: Nicht zu verwechseln ist die vorliegende Erfindung mit dem geschichtlichen "Graete-Verfahren", bei dem zur binokularen Prüfung (einer Heterophorie) zwischen dem Abbildungsstrahlengang des rechten bzw. linken Auges ca. 8cm/m vertikale Prismenwirkung geschaltet werden. Weiters gibt es außer der gängigen Polarisation zur Durchführung des binokularen Feinabgleiches Verfahren (WO2007125955) zur vertikalen Bildverdoppelung.)

Denengegenüber führt die Erfindung mehrere Abbildungsstrahlengänge unterschiedlicher Refraktion zusammen zum monokularen Vergleich. Neu und zentraler Punkt ist, dass durch geeignete technische Anordnung nur genau definierte Sehobjekte durch die zugehörigen Strahlengänge abgebildet und bei den anderen ausgelöscht werden und umgekehrt. Alle übrigen Objekte der Testfläche und des Raumes bleiben davon unbeeinflusst, erscheinen im Wesentlichen natürlich und - zum Unterschied vom geschichtlichen, wenig bekannten und kaum in Verwendung befindlichen "Bissel-Prisma" ― nicht verdoppelt.

Eine einfache technische Ausgestaltung ist durch Polarisation möglich. Dabei werden auf einem Testfeld neben nicht polarisierten Flächen Sehobjekte mit beispielsweise linearer Polarisation bei 45 bzw. 135° dargestellt. Dies können durchleuchtete Tafeln, Projektoren oder auch spezielle Flachbildschirme übernehmen. Die beiden Strahlengänge haben unterschiedliche refraktive Wirkungen und enthalten, bevor oder während sie zusammengeführt werden, entsprechend angeordnete Polfilter, sodass das eine visuelle Objekt über die diesem zugehörige refraktive Wirkung auf die Netzhaut abgebildet und gleichzeitig das andere Objekt ausgelöscht wird und umgekehrt. Das subjektive Gesamtbild bleibt natürlich und frei von Verdoppelungen.

Bei optisch-mechanischer Ausführung der Erfindung in bekannter Raumanordnung, also mit einer Bildfläche zur Darstellung der Sehobjekte in Raumentfernung und Messgläsern vor dem Auge, werden in der einfachsten Ausführung parallel unterschiedliche Strahlengänge hergestellt. Es können zwei vollständig getrennte eingerichtet werden, beispielsweise auch durch Verwendung der Linsenkombinationen des jeweiligen anderen Auges. Eine Variante ist, einen Strahlengang für die Gesamtwirkung herzustellen und nur die zu vergleichenden refraktiven Differenzen, beispielsweise nach der Kreuzzylindermethode, reell getrennt einzurichten.

In einer anderen technischen Ausführung kann ein trennendes Element gleichzeitig zwei verschiedene refraktive Wirkung enthalten, sodass von außen gar kein zweiter, gesondert geführter Strahlengang unterschieden werden kann, beispielsweise durch optische Materialien, die je nach (linearer) Polarisationsrichtung unterschiedliche Brechzahlen aufweisen.

Ein weiteres Beispiel zur technischen Realisierung sind hochfrequente Shuttersysteme. Sie müssen nur die Darstellung der Sehobjekte an subjektiv neben- oder übereinanderliegenden Orten mit der unterschiedlichen refraktiven Wirkung synchronisieren, sodass wiederum erfindungsgemäß gleichzeitig unterschiedliche Bildlagen (in der Bildweite) in Bezug auf die Netzhaut hergestellt werden. Dies ist beliebig mit der bereits oben erwähnten Aufteilung in reell-parallele Strahlengänge kombinierbar.

Die lineare Polarisation sowie die Shutter-Systeme sind nur beispielsweise angeführt weil sie derzeit die bekanntesten Trennverfahren sind. Die erfindungsgemäße Anordnung kann prinzipiell durch alle Techniken umgesetzt werden, die es erlauben, subjektiv gleichzeitig Sehobjekte mit verschiedenen Bildlagen in Bezug auf die Netzhaut auf diese abzubilden, wie etwa durch zirkulare Polarisation, Anwendung spektraler Filter oder Ähnliches.

Erfolgt eine Darstellung nicht über reelle Objekte im Raum sondern über direkte Projektion ins Auge, ist eine für diese Systeme geeignete Zuordnung von mehreren Strahlengängen zu virtuellen Sehobjekten zu gewährleisten, die dann mit unterschiedlichen Refraktionen ausgestattet werden oder es werden diese refraktiven Wirkungen durch entsprechend manipulierte Strahlengänge simuliert. Dabei können nicht nur lichtbrechende Techniken verwendet werden, sondern auch auf Diffraktion oder Interferenz beruhende sowie beliebige Kombinationen, die zur Bildentstehung beitragen und die erfindungsgemäße, gleichzeitige Erzeugung mehrerer reeller Bilder erlauben: nahe neben- oder übereinander positioniert, mit unterschiedlichen Bildlagen entlang der optischen Achse, also vor, hinter oder genau auf der Netzhaut liegend (Ziel).

Um das Wesen der Erfindung zu verdeutlichen wird nachfolgend beispielhaft eine Anwendung beschrieben, wie sie aus heutiger Sicht praktisch umsetzbar ist. Die Aufgabe besteht darin, monokular jene Refraktion zu ermitteln, bei der durch optimale Netzhautabbildung subjektiv bestes Sehen möglich ist. Dazu werden, entsprechend den gängigen, subjektiven (monokularen) Verfahren verschiedene Wirkungen in Sphäre, Zylinderstärke und Zylinderachse angeboten und in ihrer subjektiven Wirkung abgefragt. Für das erfindungsgemäße Verfahren wird keine der gebräuchlichen Frage- und Optimierungstechniken bevorzugt empfohlen, weil es für alle anwendbar ist. Trotzdem wird in weiterer Folge des öfteren vom "besten sphärischen Glas" und dem "Kreuzzylinder" die Rede sein, weil es sich dabei um Begriffe sowie Hilfsmittel für die derzeit effizienteste und genaueste Vorgangsweise handelt, eine optimale Brillenkorrektion zu ermitteln.

Bisher wurde ein "Erstes" und "Zweites" zum Vergleich zeitlich hintereinander abgefragt. Im Sinne der Erfindung werden beide Alternativen nun simultan neben- oder übereinander präsentiert. Es wird also zu einer schon vorhandenen Refraktion, die auch "plan" (= "sine correctione") sein kann, in den zweiten Strahlengang zunächst ein Plus- oder Minusglas mit sinnvoller Stärke eingebracht, sodass das zu diesem Strahlengang gehörige Objekt mit der dieser sphärischen Wirkung entsprechenden anderen Position näher oder weiter weg von der Netzhaut (Refraktionsänderung = Bildlagenverschiebung) abgebildet wird. Die Probanden geben daraufhin an, welches von den beiden als "besser" wahrgenommen wird und die Prüfer entscheiden, in welcher Weise und Schrittgröße daher weiters die sphärische Wirkung zu verändern ist, solange, bis beide als "gleich" wahrgenommen werden.

Bei Anwendung der Kreuzzylindermethode erfolgt daraufhin die Ermittlung der Achse, indem in dem einen Strahlengang die eine Position und in dem anderen die andere Position zur Wirkung gebracht wird; erfindungsgemäß sind diesen auch die jeweiligen Sehobjekte zugeordnet. Die Achse wird wiederum solange verändert, bis beide Objekte vom Probanden gleich (in diesem Fall: "gleich schlecht") beurteilt werden, worauf nach klassischer Vorgangsweise die Kreuzzylinderachse um 45° gedreht wird, um die Stärke zu ermitteln. Auch hier wird wiederum die eine Achsenlage in den einen und die um 90° gegengleiche in den anderen Strahlengang gebracht, die Zylinderstärke nach bekannter Methode je nach Antwort nachjustiert, bis wiederum beide Seheindrücke als "gleich" empfunden werden.

Der Feinabgleich der Zylinderstärke kann nach klassischer Fragetechnik ("mit oder ohne besser?") durchgeführt werden. Genau betrachtet besteht allerdings unter Anwendung geeigneter Gläser bzw. Gläserkombinationen bei der erfindungsgemäßen Anordnung kein Unterschied zwischen Grob- und Feinabgleich mehr, wenn je nach Situation zum zügigen Vorankommen zunächst große Wirkungsunterschiede angeboten werden und entsprechend auch in größeren Schritten nachgestellt wird und zum Abschluss der Proband nur mehr geringe Refraktionsdifferenzen zu vergleichen braucht, damit die besten Korrektion auch präzise fixiert wird.

Aufgrund der Einfachheit der Beurteilung durch die Probanden ist es auch möglich, dass diese die Antwort nicht verbal sondern einfach nur motorisch, durch Zeigen, Drücken, Bewegen eines Hebels oder "Joysticks" geben. Bei technischer Anordnung unter direkter Koppelung dieser motorischen Reaktion mit der Veränderung der refraktiven Wirkung in einem oder auch in beiden der Strahlengänge können Probanden direkt ihre eigene, optimale Einstellung wählen. Eine begleitende Beobachtung durch Prüfer gewährleistet sinnvolle Vorgaben (zunächst grobe, dann feine Stufung) und damit effiziente Methodik.

In den traditionellen monokularen, subjektiven Optimierungsverfahren werden als visuelle Objekte hochkontrastige Optotypen (schwarze Zeichen auf weißem Grund) verwendet. Diese Präscntationsart ist kein Abbild schwieriger, alltäglicher Sehsituationen. Die erfindungsgemäße Ausführung mit der entsprechenden optisch-technisch-elektronischen Anordnung erlaubt simultan die subjektive Beurteilung von Unterschieden, um eine optimale Korrektion für den Alltag zu ermitteln. Es dürfte also anhand von Sehobjekten mit niedrigem Kontrast, vielleicht an der Grenze der Erkennbarkeit, die Entscheidung wesentlich leichter zu treffen sein. Werden Sehobjekte mit jeweils geringem Leuchtdichtenunterschied in guter Relation mit der Größe des Refraktionsunterschiedes gewählt, ist somit das Objekt mit der einen Korrektion noch (gut) erkennbar, mit der anderen fast nicht oder auch gar nicht mehr - ist die Entscheidung einfach und klar. Dabei wird die beste Korrektion für anspruchsvolle Sehaufgaben im Alltag ermittelt, die auch die persönliche Sicherheit betreffen wic z.B. das Lenken eines Kfz bei mesopischen Leuchtdichteverhältnissen (Dämmerung, Scheinwerferlicht).

Man kann auch unterschiedliche Farben im Zusammenhang mit den Sehobjekten (ob für das Objekt selbst, ob als unterschiedliche Hintergrundfarbe oder beides) verwenden, sei es um damit ein einfaches Trennverfahren mit wechselweiser Auslöschung herzustellen, oder um die bekannte chromatische Aberration des menschlichen Auges einmal nicht als "Abbildungsfehler" zu sehen sondern mit ihr effizient zu arbeiten, wie dies gängig bereits beim sphärischen Feinabglcich mittels Rot-Grün-Test ("Bichrome-Balance-Test") geschieht. Zu beachten ist allerdings, dass die Menschen individuell auf verschiedene Farben akkommodieren, also unterschiedliche Einstellrefraktionswellenlängen haben. Zudem sind die bei Männer häufigen Dichromasien, insbesondere die Protanopie wegen der stark verminderten Empfindlichkeit für "rol", zu beachten.

Gängig werden sehr kleine Objekte zur Beurteilung von Unterschieden verwendet. Das feine Auflösungsvermögen von (hochkontrastigen) Sehaufgaben ist aber im Alltag selten nötig. So können und sollen durchaus größere Objekte, auch komplexe Bilder Verwendung finden, die dann nicht mehr nebeneinander sondern ineinander dargestellt werden - es sollen natürlich die beiden von der Schwierigkeit oder Art der Sehaufgabe her völlig ident sein. Die Trennung geschieht, optisch ― mechanisch ― elektronisch, simultan neben- oder ineinander, die qualitative Entscheidung wird durch die Probanden gefällt durch die Angabe, welches nun das besser wahrnehmbare Sehobjekt oder, an der Grenze der Erkennbarkeit, welches gut oder gerade noch sichtbar ist.

Bisher wurde von nur zwei unterschiedenen Strahlengängen mit entsprechend zugeordneten refraktiven Wirkungen gesprochen. Durch Anwendung entsprechender Technik oder gleichzeitiger Anwendung verschiedener Trennverfahren (Polarisation, Shutter, chromatische Filter, usw.) können auch drei und mehr Sehobjekte abgebildet werden, die erfindungsgemäß mit jeweils unterschiedlichen refraktiven Wirkungen bzw. Bildlagen ausgestattet sind. Beispielsweise lässt sich damit eine größere und kleinere Stärkendifferenz (z.B. beim sphärischen Wert) zum Zwecke einer zügigeren Vorgangsweise realisieren. Oder man kombiniert gleich zwei verschiedene Werte wie z.B. Achse und Zylinderstärke des zu korrigierenden Astigmatismus. Auch weitere Kombinationen, die eine zügige und effziente Vorgangsweise fördern, sind sinnvoll. Sie können hier nicht erschöpfend aufgezählt werden.

Gerade wenn mehr als zwei Möglichkeiten gleichzeitig zur Beurteilung angeboten werden, darf das die Probanden nicht verwirren. Es muss einfach und evident sein, mit welcher Reaktion die subjektive Entscheidung zu verifizieren ist. Natürlich kann dies, wie bisher gängig, durch sprachliche Begriffsbildung und verbale Kommunikation erfolgen. Demgegenüber spart aber eine rein motorische Möglichkeit durch Zeigen, Drücken entsprechender Tasten, Bewegen eines Joysticks etc., einerseits viel Zeit und Konzentrationsenergie, andererseits sind nur selten Kommunikationsfehler zu erwarten oder können gegebenenfalls schnell erkannt und korrigiert werden. Wird eine Anordnung ähnlich einem einfachen Videospiel hergestellt, ist die Motivation und Animation, nicht nur für junge Probanden, sicher höher als bisher. Neben der Beschleunigung stellt dies für die Prüfer eine deutliche Entlastung dar, sodass sie sich auf das Wesentliche bei der Betreuung konzentrieren können.

Gerade durch die beschriebene Trennung in mehr als zwei separate Strahlengänge kann die Erfindung auch mit binokularen Fragestellungen kombiniert werden. Bekanntlich sind, nachdem monokular die besten Korrektionen rechts und links bestimmt wurden, diese aufeinander abzustimmen. Dies ist nicht nur wegen des Refraktionsgleichgewichtes nötig. Es ist inzwischen bekannt, dass sich das binokulare System nicht nur in Bezug auf die Sphäre, sondern auch bezüglich der optimalen Zylinderstärke und -achse von der monokularen Korrektion signifikant unterscheiden kann, sodass die beste Korrektion natürlich nur unter binokularen Sehbedingungen, aber exakter Zuordnung der einzelnen Seheindrücke zum jeweiligen Auge, ermittelt werden kann.

Beim binokularen Sehen kommt die Ausrichtung beider Augen auf ein Sehobjekt samt den dafür notwendigen Einstell- und Fusionvorgängen dazu, die im visuellen System in starker Wechselwirkung mit Akkommodation und Einstellrefraktion, oft mit Unterschieden in Ferne und Nähe, stehen. Dies ist bekannt und wird mit unterschiedlichen Methoden analysiert und zu optimieren versucht. Hier sei nur erwähnt, dass eine erfindungsgemäße Anordnung optisch - mechanisch ― elektronischer Elemente mit allen diesen Methoden kombinierbar ist und schneller zu einem optimalen Refraktionsergebnis führt.

Nicht zuletzt kann sich durch die einfachere Entscheidungsfindung und Form der Beantwortung mit vermindertem Verwechslungspotential zwischen Probanden und Prüfern eine Atmosphäre mit weniger Stress und mehr Sicherheit entwickeln, gemeinsam ein gutes Ergebnis erarbeitet zu haben. Erfahrungsgemäß wäre dies insbesondere für die Arbeit mit Kindern, sehschwachen oder retardierten Personen wünschenswert.

## Patentansprüche

1. Verfahren zur subjektiven, monokularen Brillenglasbestimmung, **dadurch gekennzeichnet, dass** das visuelle Objekt mehrmals simultan mit unterschiedlichen refraktiven Wirkungen - Sphäre, Zylinderstärke samt Achse entsprechend - auf nahe neben- oder übereinander liegende Orte der Netzhaut abgebildet oder projiziert wird, sodass diese Darstellungen subjektiv voneinander differenziert wahrgenommen und beurteilt werden können.

2. Verfahren nach dem Anspruch 1, **dadurch gekennzeichnet, dass** die Darstellung der Zeichen über reelle Objekte, insbesondere be- und durchleuchtete Tafeln, Bildschirme, Reflexionen von Projektoren, erfolgt, mit gleichen oder derart unterschiedlichen Bildanteilen, dass bei entsprechender Zusammenführung des Abbildungsstrahlenganges dieser sowohl unterschiedliche refraktive Wirkungen erfahren als auch diesen einzelne, voneinander verschiedene visuelle Objekte zugeordnet werden.

3. Verfahren nach dem Anspruch 1, **dadurch gekennzeichnet, dass** die Entstehung des reellen Netzhautbildes über virtuelle Objekte und somit Projektion erfolgt, wobei die Strahlengänge mit den jeweiligen unterschiedlichen refraktiven Wirkungen subjektiv den verschiedenen visuellen Objekten eindeutig zugeordnet werden. Die Darstellung der refraktiven Wirkung kann auch durch entsprechend simulierend-manipulierte Strahlengänge erfolgen, die Bildentstehung auf oder in der Nähe der Netzhaut zur Gänze oder in Teilen auch durch Diffraktion sowie Interferenz bewirkt werden.

4. Verfahren nach den Ansprüchen 1 bis 3, **dadurch gekennzeichnet, dass** durch Anwendung geeigneter Trennverfahren, wie etwa Polarisation, Shutter oder chromatische Filter, einzelne visuelle Objekte jeweils in der Refraktion (gering) differierende Strahlengänge durchlaufen, ohne dass ein Objekt des Raumes doppelt erscheint.

5. Verfahren nach dem Ansprüchen 1 bis 4, **dadurch gekennzeichnet, dass** die Zusammenführung mehrerer Strahlengänge optisch-mechanisch oder opto-elektronisch getrennt von der Koppelung der jeweiligen Strahlengänge mit den zugehörigen visuellen Objekten, etwa durch Polarisation, Shutter oder chromatische Filter, stattfindet.

6. Verfahren nach dem Ansprüchen 1 bis 4, im Unterschied zu Anspruch 5 **dadurch gekennzeichnet, dass** die Zusammenführung der Strahlengänge und die Koppelung durch ein und dieselbe geeignete optisch-mechanische bzw. opto-elektronische Anordnung erfolgt.

7. Verfahren nach den Ansprüchen 1 bis 4, im Unterschied zu den Ansprüchen 5 und 6 **dadurch gekennzeichnet, dass** ein und dieselbe dafür geeignete optisch-mechanische oder optisch-elektronische Anordnung sowohl die Trennung bzw. die subjektive Zuordnung zu den visuellen Objekten ernöglicht, als auch dass diese gleichzeitig die beabsichtigte unterschiedliche refraktive Wirkung hervorruft.

8. Verfahren nach den Ansprüchen 1 bis 7, **dadurch gekennzeichnet, dass** Objekte dergestalt dargeboten, werden, dass sie nicht ausschließlich aufgrund des für die Erkennbarkeit kleinen Auflösungswinkel sondern insbesondere durch ihren geringen Kontrast an der Grenze der visuellen Wahrnehmbarkeit liegen. Wenn diese dann über unterschiedliche refraktive Wirkungen auf die Netzhaut abgebildet werden, können einzelne davon aufgrund einer gegebenenfalls suboptimalen Refraktion gar nicht wahrgenommen werden.

9. Verfahren nach den Ansprüchen 1 bis 8, **dadurch gekennzeichnet, dass** verschiedene Leuchtdichten von hell-photopisch, dunkel-photopisch, über mesopisch bis skotopisch, alltägliche oder berufliche Lichtsituationen repräsentierend, hergestellt werden, sodass das erfindungsgemäße Optimierungsverfahren je nach Adaptionszustand unterschiedliche optimale Brillenglaskorrektionen herausarbeiten kann.

10. Verfahren nach den Ansprüchen 1 bis 9, **dadurch gekennzeichnet, dass** das monokulare Prüfverfahren unter den Bedingungen der Erfindung auch binokular angewandt wird, sodass die gegebenenfalls veränderte Einstellung des visuellen Systems (durch kompensatorische Vorgänge) subjektiv beurteilt und, unter Einbeziehung insbesondere prismatischer Wirkungen, durch korrigierende Maßnahmen optimiert werden kann.

11. Verfahren nach den Ansprüchen 1 bis 10, **dadurch gekennzeichnet, dass** durch geeignete technische Anordnung eine Koppelung der verbalen oder motorischen Antwort mit der refraktiven Wirkung einzelner oder auch aller Anteile der Abbildungsvarianten erfolgt, sodass Probanden aufgrund ihrer Reaktion diese direkt, zumindest in Teilschritten, verändern und optimieren können.

12. Verfahren nach den Ansprüchen 1 bis 11, **dadurch gekennzeichnet, dass** insbesondere in Erweiterung des Anspruchs 11 durch geeignete, programmtechnische Anordnung eine automatische Führung der Probanden durch das Optimierungsverfahren erfolgt, die insbesondere die Art der Frage- bzw. Aufgabestellung, die vorgeschlagenen und zu beurteilenden Varianten, den Übergang von einzelnen Phasen bei der monokularen Optimierung in die nächste und zurück sowie den Übergang von der monokularen zur binokularen Anordnung und retour umfasst.
